# EUROPEAN PATENT APPLICATION

(11) **EP 2 495 303 A1**
(43) Date of publication of application: **05.09.2012**
(21) Application number: 10835994.4
(22) Date of filing: 08.12.2010
(51) Int. Cl.: C12M 3/00

(54) **POROUS MEMBER, METHOD FOR CAUSING POROSITY, AND METHOD FOR MANUFACTURING SAID POROUS MEMBER**

(30) Priority: 08.12.2009 JP 2009278324
(71) Applicant: JMS Co., Ltd., Hiroshima-shi Hiroshima 730-8652 (JP)
(72) Inventor: HAYASHI, Kyohei, Hiroshima 730-8652 (JP); IDE, Junichi, Hiroshima 730-8652 (JP); OGATA, Kasumi, Hiroshima 730-8652 (JP)
(74) Representative: Isarpatent
(86) International application number: PCT/JP2010/072009
(87) International publication number: WO 2011/071074

(57) **Abstract**

The present invention provides a porous member formed not by the paste method and a method of producing the porous member. The porous member of the present invention includes a core layer and a porous surface layer, wherein the core layer and the surface layer are composed of the same polymer raw material, the surface layer is formed integrally on a surface of the core layer, and the porous member does not comprise an adhesive layer between the core layer and the surface layer. Such a porous member can be produced by making the surface of a polymer substrate porous. Specifically, the porous member can be produced by immersing the polymer substrate in a solvent capable of dissolving the polymer substrate and freeze-drying the immersed polymer substrate.

## Description

### Technical Field

The present invention relates to a porous member, a porous-making method, and a method of producing the porous member. Specifically, the present invention relates to, for example, porous members serving as biomaterials such as scaffold materials for cells, stents, and the like.

### Background Art

In the fields of cell engineering and regenerative medical engineering, proliferation of cells using scaffold materials is performed for the purpose of tissue regeneration or the like. The scaffold material generally is required to have a porous structure for the purpose of maintaining disseminated cells and is also required to have appropriate rigidity for the purpose of maintaining the overall shape. However, when the scaffold material has the porous structure, flexibility is achieved but sufficient rigidity may not be achieved. Hence, in recent years, there has been a demand for providing a scaffold material having both a structural property and a physical property, i.e., the porosity and the rigidity.

As such a scaffold material, for example, a laminate of a porous member giving a porous structure and a core member giving rigidity has been proposed. As the method of producing the laminate, for example, a paste method is known (Patent Document 1). The paste method is, for example, a method of bonding the porous member and the core member to laminate by heat treatment, solvent treatment, adhesion treatment using an adhesive agent, or the like. For example, in the case of the heat treatment or the solvent treatment, the laminate is produced by melting or dissolving one surface of the porous member or the core member and bonding the both members. In the case of the adhesion treatment, the laminate is produced by applying an adhesive agent to the one surface of the porous member or the core member and bonding both the members via an adhesive agent layer.

On the other hand, in the scaffold material having a porous structure, disseminated cells are grown and proliferated within a certain thickness (for example, 100 µm order) from the surface. However, it is known that, in a region further inside than the certain thickness, even if cells are disseminated, nutrition is not sufficiently permeated thereto, and such cells finally are destroyed and hardly proliferated. Therefore, in the method of producing the aforementioned scaffold material of laminate, the thickness of the porous member is desired to be designed such that cell can be grown and proliferated therein.

### Related Art Document

### [Patent document]

[Patent document 1] US 5,514,378

### Disclosure of the Invention

### Problem to be Solved by the Invention

However, since the porous member of 100 µm order is very thin, it is difficult to handle such a porous member and it is difficult to laminate the porous member on the core member by a paste method. Particularly, it is desired to freely design the shape of the scaffold material according to a tissue to be regenerated. However, if the core member is in a complicated shape such as a tube, an auricular cartilage, or the like, it is not easy to bond the porous member to the surface of the core member having such a shape, and this is not a realistic method.

Further, in the case where the heat treatment or the solvent treatment is applied to the porous member, there is a possibility of disappearance or deformation of pores. In the case where the heat treatment or the solvent treatment is performed, an adhesive layer formed by melting or dissolving one of the members is interposed between the porous member and the core member. Further, in the case where the adhesion treatment is performed, the adhesive layer composed of the adhesive agent is interposed between the porous member and the core member. Such adhesive layers are unnecessary for a function of the scaffold material.

These problems are not limited to the scaffold material but also applied to biomaterials such as an adhesion-preventing material and a stent, which attract attention as the use of the porous member.

Hence, the present invention is intended to provide, for example, a porous member formed not by the paste method; a method of making a polymer substrate porous for producing the porous member; and a method of producing the porous member.

### Means for Solving Problem

The porous member of the present invention is a porous member having a porous surface. The porous member includes a core layer and a porous surface layer, wherein the core layer and the surface layer are composed of the same polymer raw material, the surface layer is formed integrally on a surface of the core layer, and the porous member does not include an adhesive layer between the core layer and the surface layer.

The porous-making method of the present invention is a method for making a surface of a polymer substrate porous. The method includes the following processes (A) and (B): (A) immersing the polymer substrate in a solvent capable of dissolving the polymer substrate; and (B) freeze-drying the immersed polymer substrate.

The production method of the present invention is a method of producing a porous member, wherein a surface of a polymer substrate is made porous by the porous-making method of the present invention.

The scaffold material of the present invention is produced by the production method of the present invention.

### Effects of the Invention

The porous member of the present invention is a porous member not formed by the paste method. Therefore, unlike the porous member obtained by the paste method, for example, the porous member of the present invention does not include an adhesive layer formed by bonding between the core layer and the porous surface layer. Such a porous member can be produced, for example, by the porous-making method and the production method of the present invention. In other words, according to the porous-making method and the production method of the present invention, the surface of the polymer substrate can be made porous easily by simply immersing the polymer substrate in the solvent and then freeze-drying the polymer substrate. In this manner, the porous member of the present invention in which the surface layer is integrally formed on a porous surface of the core layer can be produced.

Further, according to the porous-making method and the production method of the present invention, as described above, a porous surface layer can be formed simply by immersing the polymer substrate in the solvent and freeze-drying the immersed polymer substrate. Therefore, according to the production method of the present invention, the porous member including the porous surface layer and the core layer as a single member can be formed easily regardless of the shape of the polymer substrate. In this manner, since a desired shaped porous member that does not include an adhesive layer can be produced easily according to the present invention, the range of application of the porous member can be increased. Therefore, the present invention is very effective in the field of regenerative medicine, for example.

### Brief Description of Drawings

[FIG. 1] FIG. 1 is a cross sectional photograph of an example of the scaffold material in Example 1 of the present invention.
[FIG. 2] FIG. 2(A) is a graph showing the relationship between the immersion time and the thicknesses of the surface layers; and FIG. 2(B) is a graph showing the relationship between the immersion time and the thickness of the core layer in Example 1.
[FIG. 3] FIG. 3 is a scanning electron micrograph of an example of the scaffold material in Example 1 of the present invention.
[FIG. 4] FIG. 4 is a graph showing the relationship between the freezing treatment temperature and the average pore size of the surface layer in Example 1 of the present invention.
[FIG. 5] FIG. 5 is a graph showing the result of measurement of the number of cells in Example 2 of the present invention.
[FIG. 6] FIG. 6 is a photograph of the appearance of the auricle shaped scaffold material in Example 3 of the present invention.
[FIG. 7] FIGs. 7(A) and 7(B) are cross sectional photographs of the auricle shaped scaffold material in Example 3 of the present invention.
[FIG. 8] FIGs. 8(A) to 8(C) show an example of the porous member of the present invention: FIG. 8(A) is a plan view; FIG. 8(B) is a cross sectional view; and FIG. 8(C) is a perspective view in use.
[FIG. 9] FIGs. 9(A) and 9(B) show another example of the porous member of the present invention: FIG. 9(A) is a perspective view; and FIG. 9(B) is a cross sectional view.

### Description of Embodiments

### <Porous member>

As described above, the porous member of the present invention is a porous member having a porous surface. The porous member includes a core layer and a porous surface layer, wherein the core layer and the surface layer are composed of the same polymer raw material, the surface layer is integrally formed on the surface of the core layer, and the porous member does not include an adhesive layer between the core layer and the surface layer. It also can be said that, in the present invention, the surface layer is formed on the surface of the core layer without involving the adhesive layer, for example.

The porous member of the present invention can be produced, for example, by the production method that will be described below. The reason why the foregoing porous member can be produced by the production method of the present invention can be presumed as follows. When the polymer substrate is immersed in the solvent, the surface of the polymer substrate is dissolved gradually by the solvent and swelled by the solvent, and becomes, for example, in a gel form. In this state, when the polymer substrate is freeze-dried, the swelled region at the surface of the polymer substrate is made porous, and as a result, the porous member including the polymer substrate that has a porous surface can be obtained. That is, in the polymer substrate, the surface of the polymer substrate that is made porous after swelling serves as the porous surface layer and the region of polymer substrate that is not swelled serves as the core layer. Therefore, the porous member including the porous surface layer and the core layer as a single member and not including the adhesive layer between the both layers can be formed. In the porous member of the present invention, it can be said that the core layer is a layer that maintains the physical properties of the polymer substrate, for example. Note here that the present invention is not limited at all to this presumption.

In the present invention, the terms, "integrally formed" and "as a single member", mean that the core layer and the surface layer are not bonded as separated members. Therefore, as described above, the porous member of the present invention does not include the "adhesive layer", which is formed by bonding, between the core layer and the porous surface layer. The "adhesive layer" is a layer formed by separately providing the core member and the porous member and bonding them as in the paste method, for example. An example of the "adhesive layer" includes a layer containing the adhesive agent (adhesive agent layer) in the case where the core member and the porous member are bonded using an adhesive agent. Specific examples thereof include a layer formed between the core member and the porous member and a layer formed by the introduction of the adhesive agent into pores exposed at the bonding surface of the porous member or the core member. Further, in the case where the core member and the porous member are bonded by the heat treatment or the solvent treatment, an example of the "adhesive layer" includes a layer formed by dissolving or melting the core member or the porous member and then solidifying it. A specific example in the case where the core member is dissolved or melted includes a layer formed by introducing a polymer that is dissolved or the like into pores exposed at the bonding surface of the porous member and then solidifying it. Further, a specific example in the case where the porous member is dissolved or melted includes a layer formed by the disappearance, the reduction, or the deformation of pores at the bonding surface of the porous member due to dissolution or the like of the polymer.

There is no particular limitation on the application of the porous member of the present invention and examples thereof include biomaterials used in vivo and non-biomaterials used in vitro. Examples of the biomaterials include scaffold materials for cells and tissues in vivo, adhesion-preventing materials, stents, and bioprostheses. There is no particular limitation on the organism to which the biomaterial is applied and examples thereof include animals such as human, mammals excluding human, and birds. Examples of the mammals excluding human include monkeys, dogs, cats, horses, cattle, sheep, goats, pigs, mice, rats, rabbits, and hamsters. Examples of the non-biomaterials include scaffold materials for cells and tissues collected from organisms; substrates such as cell culture containers; and the like.

There is no particular limitation on the size of the porous member of the present invention and the size can be set suitably according to the intended use.

There is no particular limitation on the shape of the porous member and the shape can be set suitably according to the intended use, for example. Specifically, the porous member may be in a shape of a film, a plate, a block, a column, a cylinder, a tube, a sphere, a cone, a pyramid, or the like. In addition, a shape of the combination of these shapes may be employed. The porous member may be a hollow member or a non-hollow member. The porous member may take the entire or a partial shape of a biological tissue, for example. There is no particular limitation on the biological tissue and examples thereof include the auricle, nose, menisci, pharyngeal vault, eyes, teeth, blood vessels, bones, cartilage, ligament, skin, breasts, tail, spinal cord, brain, pancreas, liver, kidneys, heart, and intestines.

In the porous member of the present invention, the surface layer may be formed on the entire surface of the core layer or may be formed on a partial surface of the core layer. The region of the surface of the core layer on which the surface layer is formed can be suitably decided according to the intended use of the porous member, for example. In the case where the porous member is used in vivo, preferably, the surface layer is formed on the surface of the porous member at the region that requires the affinity with cells in vivo, for example.

There is no particular limitation on the thickness of the surface layer, and is, for example, from several tens µm order to several hundreds µm order, preferably from 10 to 1,000 µm, more preferably from 50 to 500 µm, and yet more preferably from 100 to 150 µm.

Preferably, the thickness of the surface layer is substantially uniform, for example. "The thickness of the surface layer is substantially uniform" refers to, for example, the case where the variation in the thickness of the surface layer is, for example, from ±0% of the average thickness to ±15% of the average thickness and preferably from ±0% of the average thickness to ±5% of the average thickness. The variation includes, for example, a standard deviation of the thickness measured.

There is no particular limitation on the size and the thickness of the core layer, and the size and the thickness can be decided suitably according to, for example, the intended use of the porous member, the rigidity required for the porous member, and the like. For example, the thickness of the core layer is preferably larger than that of the surface layer. Specifically, the thickness is, for example, 1,000 µm or more.

There is no particular limitation on the pore size of the surface layer, and the pore size can be decided suitably according to the intended use of the porous member, for example. The average pore size of the surface layer is, for example, 5 to 500 µm and preferably 10 to 60 µm. The pore size of the surface layer is preferably uniform, although it can be ununiform, for example.

The core layer may be porous or non-porous, for example. In the case where the core layer is porous, there is no particular limitation on the average pore size and is, for example, 1 to 10 µm. The average pore size of the core layer may be uniform or ununiform, for example, and is not particularly limited.

There is no particular limitation on the porosity of the surface layer, and the porosity is, for example, 50 to 99%.

In the case where the core layer is porous, there is no limitation on the porosity. Preferably, the porosity of the core layer is relatively lower than that of the surface layer, for example, for maintaining the rigidity. Specifically, the porosity of the core layer is, for example, less than 50%.

The porous member of the present invention further may include other layer(s), for example. The other layer may be one layer or two or more layers, for example. In the case where the porous member of the present invention includes other layer, the other layer is preferably laminated on the core layer at the region on which the surface layer is not formed, for example. At this time, the other layer and the core layer may be laminated via the adhesive layer.

The porous member of the present invention may include a pharmaceutical preparation, for example. There is no particular limitation on the pharmaceutical preparation, and examples thereof include bioactive substances such as various proliferators; anti-infective agents; anticancer agents; anti-inflammatory agents; and analgesic agents.

Hereinafter, examples of the application of the porous member of the present invention will be described. Note here that the present invention is not limited at all to these examples.

### (1) Scaffold material

The scaffold material is a member serving as a scaffold for cell proliferation or tissue proliferation, for example. The scaffold material may be used in vitro or in vivo, for example. In the case where the scaffold material is used in vitro, for example, by disseminating and culturing cells or tissues, the scaffold material can grow or proliferate the cells or tissues. The cells may be cells collected from organisms or cultured cell strains, for example. There is no particular limitation on the type of the cell, and examples thereof include blood vessel cells, cartilage cells, ß cells of islets of Langerhans, skin cells, neuronal cells, mesenchymal stem cells, osteoblasts, and adipocytes. There is no particular limitation on the origin of the cell, and examples thereof include the aforementioned animals. The scaffold material in which cells are cultured in this manner may be placed in vivo after culturing cells, for example. On the other hand, in the case where the scaffold material is used in vivo, for example, the scaffold material is placed in a tissue in vivo and can proliferate cells and tissues in vivo. There is no particular limitation on the biological tissue in which the scaffold material is placed, and examples thereof include the aforementioned biological tissues. Further, there is no particular limitation on the type of living subject to which the scaffold material is applied, and examples thereof include the aforementioned animals.

There is no particular limitation on the shape of the scaffold material and the scaffold material is, for example, in a shape of a block. In the case of the scaffold material, the pore size of the surface layer is preferably the size that allows cells to be disseminated and proliferated. The average pore size of the surface layer is, for example, 5 to 500 µm and preferably 10 to 60 µm.

### (2) Adhesion-preventing material

The adhesion-preventing material is a member that prevents adhesion between tissues by placing it between the tissues in vivo, for example.

There is no particular limitation on the shape of the adhesion-preventing material, and the adhesion-preventing material is preferably in a shape of a film or a sheet. With respect to the adhesion-preventing material, for example, the core layer preferably has the porous surface layer only at one side, although the core layer may have the porous surface layers at the both sides.

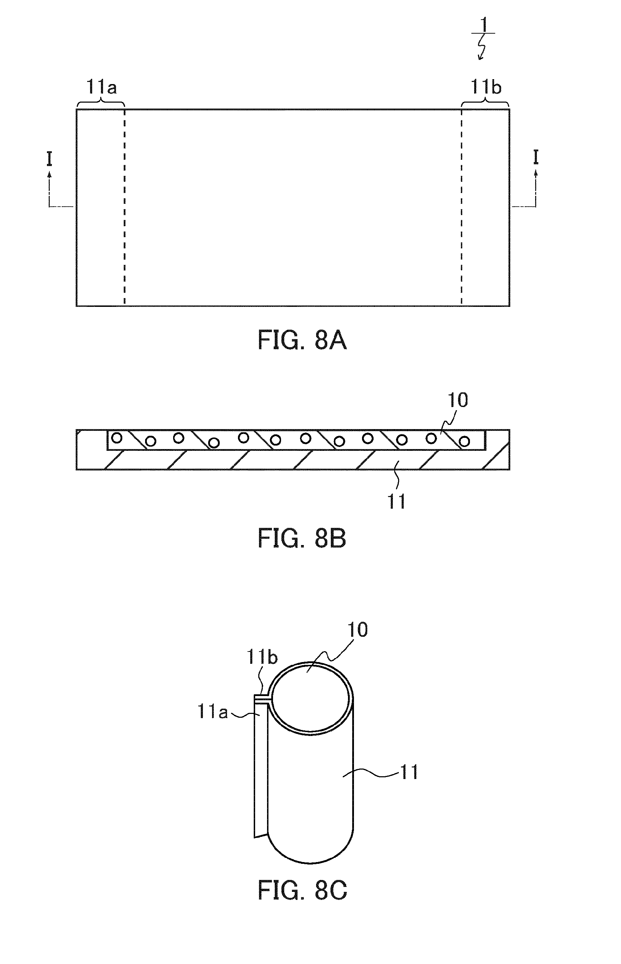

The adhesion-preventing material may directly be placed in an affected area in vivo, for example. In the case where the affected area is in a tubular shape, the adhesion-preventing material may wind around the affected area, for example. In the case where the adhesion-preventing material is used in a winding state, for example, the porous member shown in FIGs. 8(A) to 8(C) is preferable. FIGs. 8(A) to 8(C) show an example of the porous member of the present invention. FIG. 8(A) is a plan view of a porous member 1, FIG. 8(B) is a cross sectional view taken along the line I-I of the porous member 1 shown in FIG. 8(A), and FIG. 8(C) is a perspective view of the porous member 1 in use. The porous member 1 includes a core layer 11 and a porous surface layer 10. The surface layer 10 is formed on the surface of the core layer 11 except for both end portions 11a and 11b of the core layer 11 without involving the adhesive layer. The porous member 1 can be used, for example, as shown in FIG. 8(C). That is, the porous member 1 winds around the affected area such that the porous surface layer 10 is in contact with the tubular affected area. Then, the end portions 11a and 11b of the core layer 11 are overlapped with each other and sealed using an electric scalpel or the like. Since the end portions 11a and 11b to be sealed are intended to be sealed firmly, for example, the core layer 11 is preferably non-porous rather than porous.

### (3) Stent

The stent is generally a tubular device placed in tubular biological tissues such as the blood vessel, trachea, esophagus, duodenum, and bile duct. For example, by inserting the tubular stent into the lumen of the tubular tissue, constriction or the like due to anastomosis can be prevented.

Since the stent is used by insertion into the lumen as described above, the stent is preferably in a shape of a tube, for example. Further, since the stent is used in the lumen, the outer surface of the stent is in contact with the tubular tissue, and bile, blood, digesta, or the like passes through the inside of the stent according to the type of the tubular tissue, for example. For stably placing the stent in vivo, for example, it is desired that the outer surface of the stent has a high affinity with biological cells. Therefore, the stent is preferably a porous member in which the porous surface layer is formed on its outer surface. When such a porous member is used as a stent, for example, in vivo, cells in vivo which are in contact with the outer surface are introduced into the surface layer of the stent and proliferated. Therefore, the stent can be placed stably. On the other hand, since bile or the like passes through the inside of the stent as described above, for example, the stent does not need to have the porous surface layer at the inside and the inner surface of the stent may be a non-porous surface layer, for example.

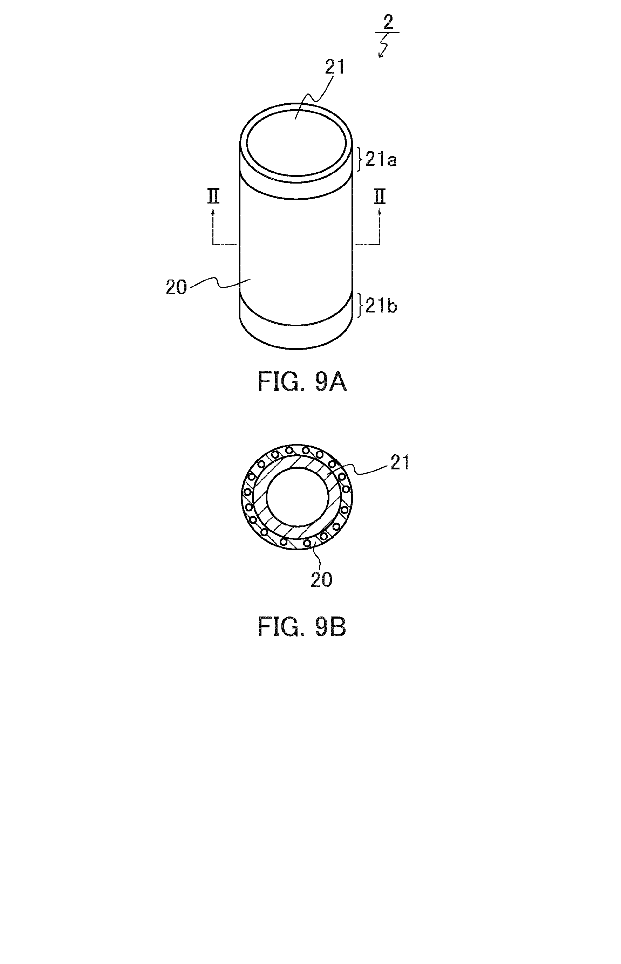

For example, the stent is preferably the porous member shown in FIGs. 9(A) and 9(B). FIGs. 9(A) and 9(B) show an example of the porous member of the present invention. FIG. 9(A) is a perspective view of a porous member 2 and FIG. 9(B) is a cross sectional view taken along the line II-II of the porous member 2 in FIG. 9(A). The porous member 2 includes, for example, a tubular core layer 21 and a tubular surface layer 20. The surface layer 20 is integrally formed on the surface of the core layer 21 except for both end portions 21a and 21b of the core layer 21. With respect to the porous member 2, each of the end portions 21a and 21b is inserted into the lumen and is sutured with the tubular tissue. Since it is desired that the both end portions 21a and 21b to be sutured are sutured without being destroyed, for example, each of the end portions 21a and 21b is preferably non-porous rather than porous, and more preferably is the non-porous core layer having a higher thread tension than the porous surface layer.

### (4) Bioprosthesis

The bioprosthesis is a member for supplementing cells in vivo or tissues in vivo by placing it at a defect site in vivo. Specific examples of the bioprosthesis include artificial bones to be placed at defect sites of bones and bone pins for fixing bones.

### <Porous-making method>

The porous-making method of the present invention is a method for making the surface of a polymer substrate porous. The method includes the following processes (A) and (B): (A) immersing the polymer substrate in a solvent capable of dissolving the polymer substrate; and (B) freeze-drying the immersed polymer substrate.

According to the foregoing method, the surface of the polymer substrate can be made porous. Therefore, the aforementioned porous member of the present invention can be produced. The porous-making method of the present invention can also be referred to as a surface modification method of a polymer substrate, for example.

### (A) Immersion process

The size and the shape of the porous member of the present invention depend on the size and the shape of the polymer substrate to be used, for example. There are no particular limitations on the size and the shape of the polymer substrate, and the size and the shape can be decided suitably according to the size and the shape of the desired porous member, for example. Examples of the shape of the polymer substrate include the examples of the shape described for the porous member. The polymer substrate may be a hollow substrate or a non-hollow substrate. Further, the shape of the polymer substrate may take the entire or a partial shape of a biological tissue, for example. There is no particular limitation on the biological tissue, and examples thereof include the examples of the biological tissue described above.

The polymer substrate may be porous or non-porous, for example. In the present invention, "porous-making" includes the following meaning. That is, in the case where the surface of the polymer substrate before immersion treatment is porous, the surface of the polymer substrate is made further porous, e.g., the diameters of pores are reduced or increased or the number of pores is increased.

The polymer substrate may be, for example, a single layer polymer substrate or a multilayer polymer substrate made of two or more layers. In the latter case, for example, the respective layers may be composed of the same polymer raw material or different polymer raw materials. With respect to the multilayer polymer substrate, the physical properties such as rigidity and a degradation rate in vivo can be set according to a request, for example, by appropriately selecting the polymer raw material of each layer.

There is no particular limitation on the polymer raw material that constitutes the polymer substrate, and various polymers can be employed. Further, there is no particular limitation on the polymer. In the case where the porous member is used in vivo, the polymer is preferably a polymer that shows biocompatibility, for example. Further, a biodegradable polymer that degrades in vivo after the elapse of a certain period of time may be used. In the case where the porous member is used in vitro or in the case where the porous member is semipermanently kept in vivo, the polymer may be, for example, a non-biodegradable polymer.

There is no particular limitation on the molecular weight of the polymer and the molecular weight is, for example, 5,000 to 2,000,000, preferably 10,000 to 1,500,000, and more preferably 100,000 to 1,000,000. The polymer can be, for example, a homopolymer or a copolymer such as a random polymer, a block polymer, or a graft polymer.

There is no particular limitation on the biodegradable polymer, and examples thereof include aliphatic polyester, polyvinyl alcohol, polyethylene glycol, polycarbonate, and polyamide. Among them, aliphatic polyester is preferable. There is no particular limitation on the monomer that constitutes the biodegradable polymer, and examples thereof include lactic acid, lactide, lactone, glycolide, glycolic acid, trimethylene carbonate, ethylene carbonate, diisocyanate, para-dioxanone, and ethylene oxide. Further, a copolymer obtained by combining these monomers may be employed. Examples of the lactone include γ-butyrolactone, δ-valerolactone, and ε-caprolactone; and ε-caprolactone is preferable. In the case where the biodegradable polymer is a copolymer, there are no particular limitations on the combination and the ratio of the monomers. Examples of the combination of the monomers include the combination of lactide and lactone and the combination of glycolide and lactone. Among them, the combination of lactide and lactone is preferable, and specific examples of the combination include the combination of lactide and ε-caprolactone and the combination of glycolide and ε-caprolactone. Among them, the combination of lactide and ε-caprolactone is preferable. There is no particular limitation on the ratio of the monomers to be combined. In the case of the copolymer obtained by combining lactide and lactone (for example, ε-caprolactone), the molar ratio (lactide:lactone) is, for example, 90:10 to 10:90, preferably 85:15 to 20:80, and more preferably 80:20 to 40:60.

The biodegradable polymer may be, for example, a natural polymer such as collagen, hyaluronic acid, elastin, chitosan, chitin, chondroitin sulfate, or cellulose. There is no particular limitation on the natural polymer and examples thereof include extracts from biological tissues, biological cells, and the like; products obtained by transformant; and complexes. The natural polymer may be, for example, the one obtained by further modifying or derivatizing the extract, the product, or the complex.

There is no particular limitation on the non-biodegradable polymer, and examples thereof include polyethylene and polyurethane.

The polymer raw material may contain one of the aforementioned polymers or two or more of the aforementioned polymers, for example. In the latter case, there are no particular limitations on the combination and the ratio thereof, and the combination and the ratio can be set appropriately.

The polymer raw material may contain other component(s) besides the aforementioned polymer, for example. There is no particular limitation on the other component(s), and the polymer raw material may contain hydroxyapatite, titanium, and the like, for example.

There is no particular limitation on the solvent in which the polymer substrate is immersed, and solvents capable of dissolving the polymer substrate can be used. Specifically, in the case where the polymer substrate is the single layer polymer substrate, as the solvent, a solvent capable of dissolving the single layer polymer substrate can be used, i.e., a solvent capable of dissolving the polymer raw material that constitutes the single layer polymer substrate can be used. In the case where the polymer substrate is the multilayer polymer substrate, as the solvent, a solvent capable of dissolving at least one of the outermost layers, i.e., the surface(s) thereof that is to be made porous, can be used. That is, a solvent capable of dissolving the polymer raw material(s) that configures the outermost layer(s) can be used.

The solvent can be decided suitably according to the type or the like of the polymer raw material, for example. Specific examples of the solvent include organic solvents such as acetone, toluene, benzene, chloroform, methyl ethyl ketone, 1,4-dioxane, dimethyl carbonate, dimethylformamide, and hexafluoroisopropanol; and aqueous solvents such as water and the like. For example, one of the solvents may be used alone or a mixed solvent of two or more solvents may be employed. The mixed solvent may be a mixed solvent of the organic solvent and the aqueous solvent, for example. There is no particular limitation on the combination of the solvent and the polymer raw material. Specific examples of the combination include the combination of 1,4-dioxane and lactide-caprolactone copolymer, the combination of hexafluoroisopropanol and polyglycolic acid, and the combination of hexafluoroisopropanol and poly(para dioxanone).

There is no particular limitation on the time for immersing the polymer substrate in the solvent, and the time can be decided suitably according to the combination of the polymer substrate and the solvent. The immersion time is, for example, 1 to 600 seconds. In the production method of the present invention, for example, the thickness of the surface layer to be formed, the pore size of the surface layer to be formed, and the like can be controlled by adjusting the immersion time. Specifically, for example, when the immersion time is set relatively long, the thickness of the surface layer can be relatively increased and the pore size of the surface layer can be relatively increased. On the other hand, for example, when the immersion time is set relatively short, the thickness of the surface layer can be relatively decreased and the pore size of the surface layer can be relatively decreased. In the production method of the present invention, as described above, the swelled region of the polymer substrate serves as the surface layer and the non-swelled region serves as the core layer. Therefore, for example, the thickness of the core layer decreases as the thickness of the surface layer increases.

There is no particular limitation on the immersion treatment temperature. For example, the immersion treatment temperature is preferably higher than the melting point of the solvent for immersion and lower than the boiling point of the solvent for immersion. In the case where 1,4-dioxane is used as the solvent, the immersion treatment temperature is, for example, in the range from 12 to 101°C.

The immersion of the polymer substrate can be performed by immersing the polymer substrate in the container filled with the solvent. Preferably, the polymer substrate is immersed in the solvent in the state where it is not in contact with the side surfaces and the bottom surface of the container, for example. Thereby, for example, the porous surface layer can be formed more uniformly. Further, at the time of immersing the polymer substrate in the solvent, for example, the solvent in the container may be stirred gently with a stirrer or the like.

In the case where the polymer substrate is the single layer polymer substrate as described above, for example, the polymer substrate may be immersed entirely or partially in the solvent. In the case of making the entire surface of the single layer polymer substrate porous, preferably, the single layer polymer substrate is immersed in the solvent with the entire surface thereof being exposed. In the case of making a desired surface of the single layer polymer porous, for example, only the desired surface may be immersed in the solvent or the entire single layer polymer substrate may be immersed in the solvent with only the desired surface being exposed. There is no particular limitation on the method of exposing only a desired surface and, for example, this can be performed by masking an arbitrary surface of the polymer substrate. In the case where the single layer polymer substrate is used, for example, a non-porous part of the polymer substrate serves as the core layer of the porous member of the present invention and a porous surface serves as the surface layer of the porous member of the present invention.

In the case where the polymer substrate is the multilayer polymer substrate as described above, for example, the polymer substrate may be immersed entirely or partially in the solvent. In the case where the both outermost layers of the multilayer polymer substrate are capable of being dissolved in the solvent, for example, immersion treatment can be set depending on whether making only one of the surfaces porous or making both of the surfaces porous. In the case of making only one of the surfaces porous, only one of the outermost layers of the multilayer polymer substrate may be immersed in the solvent or the entire multilayer polymer substrate may be immersed in the solvent with only one surface of the outermost layers being exposed. Further, in the case of making both of the surfaces porous, for example, the entire multilayer polymer substrate may be immersed in the solvent. In the case where one of the outermost layers of the multilayer polymer substrate is capable of being dissolved in the solvent and the other of the outermost layers is not capable of being dissolved in the solvent, for example, the entire multilayer polymer substrate may be immersed in the solvent.

In the case where the polymer substrate is the multilayer polymer substrate and only one of the outermost layers is made porous, preferably, the one of the outermost layers of the multilayer polymer substrate is a layer capable of being dissolved in the solvent and the other of the outermost layers is a layer not capable of being dissolved in the solvent. With such a multilayer polymer substrate, for example, only a desired surface can be made porous easily utilizing the difference between the solubilities of the respective layers to the solvent by simply immersing the entire substrate in the solvent. The layer capable of being dissolved in the solvent and the layer not capable of being dissolved in the solvent can be set, for example, based on the solubility of the polymer raw material relative to the solvent. As a specific example, a layer composed of a copolymer of lactide and caprolactone (P (LA/CL)) and a layer composed of a polyglycolic acid (PGA) can be employed. For example, the multilayer polymer substrate with the aforementioned layers as outermost layers is immersed in dioxane capable of dissolving P (LA/CL). In this case, since PGA is not dissolved in dioxane, only the outermost layer of P (LA/CL) is made porous and the outermost layer of PGA is not made porous. In the case where the multilayer polymer substrate is a multilayer polymer substrate of three or more layers, there is no particular limitation on the type of an intermediate layer interposed between two outermost layers.

Here, "not being dissolved in the solvent" includes the meaning that the substrate is not virtually dissolved in the solvent, for example, in addition to the meaning that the substrate is not completely dissolved. "The substrate is not virtually dissolved" means that the substrate may be dissolved but the dissolution does not correspond to the dissolution of making the substrate porous, which is the purpose of the present invention.

In the case where the multilayer polymer substrate is used, for example, the non-porous part of the outermost layer serves as the core layer of the porous member of the present invention and the porous part of the outermost layer serves as the surface layer. Also in the case of using the multilayer polymer substrate, as in the case of using the single layer polymer substrate, for example, by masking an arbitrary surface of the outermost layer, only a desired surface can be exposed and can be made porous.

Specific examples of the method of masking in the porous-making method of the present invention will be described hereinafter. However, the present invention is not limited at all thereto.

In the case where the porous member shown in FIG. 8 is produced, for example, the parts of the film-shaped polymer substrate corresponding to the end portions 11a and 11b shown in FIG. 8 and the entire area of one of the surfaces are subjected to masking. There is no limitation at all on the member used for masking as long as the solvent is not penetrated into the regions that have been masked, for example. The entire polymer substrate is immersed in the solvent in the state where the polymer substrate is masked. Thereby, the surface of the polymer substrate except for the regions that have been masked is made porous. The porous surface made in this manner corresponds to the surface layer 10 in FIG. 8.

In the case where the tubular porous member shown in FIG. 9 is produced, for example, a columnar substrate composed of a material (for example, silicon) insoluble to a solvent is inserted into the hollow of the tubular polymer substrate, and the parts of the polymer substrate corresponding to the end portions 21a and 21b shown in FIG. 9 are subjected to masking, i.e., the both ends of the outer surface of the polymer substrate are subjected to masking. The entire polymer substrate is immersed in the solvent in the state where the polymer substrate is masked. Thereby, the surface of the polymer substrate except for the regions that have been masked is made porous, i.e., the region except for the inner surface of the hollow and the both ends of the outer surface is made porous. The porous surface made in this manner corresponds to the surface layer 20 in FIG. 9.

### (B) Freeze-drying process

Next, the polymer substrate immersed in the solvent is subjected to freeze-drying. Normally, freeze-drying can be performed by drying the immersed polymer substrate under reduced pressure after freezing treatment is applied thereto.

There is no limitation on the freezing treatment temperature, and is, for example, -196 to 0°C and preferably -50 to 0°C. The freezing treatment temperature may be, for example, the preset temperature of a freezer at the time when the polymer substrate is frozen by cooling or the temperature of the polymer substrate at the time of the freezing treatment and/or at the time of the completion of freezing. The production method of the present invention can control, for example, the pore size of the surface layer to be formed by adjusting the freezing treatment temperature. Specifically, for example, when the freezing treatment temperature is set relatively high, the pore size can be set relatively large. On the other hand, when the freezing treatment temperature is set relatively low, the pore size can be set relatively small.

For example, cooling of the polymer substrate may be performed under constant temperature or may be performed by gradually decreasing the freezing treatment temperature. In the latter case, the freezing treatment temperature may be intermittently decreased or continuously decreased. By setting the cooling rate constant, a surface layer having pores of relatively uniform pore sizes can be formed, for example. In the case where the freezing treatment temperature is continuously decreased, for example, the temperature may be continuously decreased at a constant cooling rate. There is no particular limitation on the cooling rate and is, for example, -3 to -1,000 °C/hour and preferably -3 to -750 °C/hour. In the case where the freezing treatment temperature is decreased from a freezing start temperature to a freezing completion temperature, the freezing completion temperature (final freezing treatment temperature) is, for example, within the aforementioned temperature range.

There is no particular limitation on the freezing treatment time from the cooling start to the cooling completion. The freezing treatment time is, for example, 0.1 to 5 hours.

The immersed polymer substrate may be subjected to freezing treatment in the state where it is immersed in the solvent or may be subjected to freezing treatment in the state where it is taken out from the solvent, and the former is preferable. In the case where the polymer substrate is subjected to freezing treatment in the state where it is immersed in the solvent, for example, the freezing treatment can be performed while maintaining the shape of the swelled region at the surface of the polymer substrate formed in the solvent. Therefore, for example, the decrease in the uniformity of the surface layer to be formed further can be prevented.

There is no particular limitation on the means for freezing, for example, and conventionally known equipment such as a freezer can be used.

At the time of drying the frozen body of the polymer substrate, there is no particular limitation on the temperature for the reduced pressure drying treatment, and is, for example, -50 to 90°C and preferably -20 to 25°C. The temperature for the reduced pressure drying treatment is, for example, the preset temperature of a dryer at the time when the polymer substrate is subjected to the reduced pressure drying treatment. There is no particular limitation on the time for the reduced pressure drying treatment as long as, for example, the amount of the solvent contained in the frozen body of the polymer substrate obtained in the process (B) can be reduced, i.e., the solvent can be removed from the frozen body. The treatment time is, for example, 0.5 to 120 hours and preferably 1.5 to 12 hours. There is no particular limitation on the pressure set in the reduced pressure drying and is, for example, 1 to 2 Pa. In the present invention, "reduced pressure" includes the meaning of vacuum, for example.

The frozen body may be dried under constant temperature or may be dried by gradually increasing the treatment temperature, for example. In the latter case, for example, the treatment temperature may be intermittently increased or continuously increased. In the latter case, for example, the temperature may be continuously increased at a constant rate of temperature increase. There is no particular limitation on the rate of temperature increase and is, for example, 1 to 150 °C/hour and preferably 6.25 to 50 °C/hour. In the case where the treatment temperature is increased, the final treatment temperature is, for example, within the aforementioned temperature range. The production method of the present invention can control the pore size of the surface layer and can form a surface layer having pores of relatively uniform pore sizes, for example, by adjusting the rate of temperature increase.

There is no particular limitation on the means for drying, for example, and conventionally known equipment such as a freeze-dryer can be used.

In this manner, the aforementioned scaffold materials of the present invention can be produced by immersing the polymer substrate in the solvent in the process (A) and freeze-drying in the immersed polymer substrate in the process (B).

### <Production method>

The production method of the present invention is a method of producing a porous member characterized by making the surface of the polymer substrate porous by the porous-making method of the present invention. Specifically, the present invention is, for example, a method of producing a porous member that has a porous surface. The method includes the following processes (A) and (B): (A) immersing the polymer substrate in a solvent capable of dissolving the polymer substrate; and (B) freeze-drying the immersed polymer substrate.

The production method of the present invention is characterized by performing the porous-making method of the present invention, and other processes and conditions are not limited at all. The production method of the present invention can be performed by referring to the porous-making method of the present invention, unless otherwise noted.

Hereinafter, the present invention will be described in detail with reference to Examples. However, the present invention is not limited thereto.

### Examples

### (Example 1)

In Example 1, scaffold materials having different surface layer thicknesses were produced by changing the immersion time.

### (Production of polymer substrate)

First, lactide-caprolactone copolymer P (LA/CL) was provided. In the P (LA/CL), the molar ratio between L-lactide and ε-caprolactone after synthesis was 75.1:24.9 and the average molecular weight (Mw) was 351,000. In a stainless steel mold having an outer diameter of 120 µm, an inner diameter of 100 µm, and a cavity depth of 1,000 µm, a Teflon (registered trademark) sheet (trade name: NITOFLON No.970-2UL) and a Teflon (registered trademark) film (trade name: NITOFLON No.900UL) were placed in this order, and then the P (LA/CL) powder was uniformly placed thereon. Then, on the powder, the Teflon (registered trademark) film and the Teflon (registered trademark) sheet of the same types as above were laminated in this order, and an upper plate was placed thereon. A pressing plate was placed on the upper plate. Thereafter, the powder in the mold was pressed at 170°C and 0.5 MPa for 5 minutes, and then the pressing plate was moved up and down once (1 second for each) for defoaming. Further, the resultant was pressed at 170°C and 10 MPa for 5 minutes, and thereafter, it was water-cooled with the mold to 40°C under pressing. The obtained molded polymer having a thickness of 1,000 µm was cut into 10 mm × 20 mm pieces, and each piece was used as a polymer substrate.

### (Production of scaffold material)

About 50 mL 1,4-dioxane (produced by Wako Pure Chemical Industries, Ltd.) was poured into a metallic Schale (diameter: 5 cm), and the polymer substrate was immersed therein for a predetermined period (2, 10, 60, 120, 180, 300, or 600 seconds). After immersion, the metallic Schale was disposed on a cooling rack set at a predetermined temperature provided in a freeze-dryer in the state where the polymer substrate is immersed therein, and the metallic Schale was cooled to freeze the polymer substrate. The temperature of the cooling rack was regarded as the freezing treatment temperature and was set at a constant predetermined temperature (0°C, -30°C, or -50°C). In the case where the temperature of the cooling rack was 0°C, the cooling time was 3 hours; and in the case where the temperature of the cooling rack was ―30°C or -50°C, the cooling time was 1 hour. After freezing, the temperature in the freeze-dryer was increased to 25°C at a rate of temperature increase of 25 °C/hour to conduct vacuum drying, and thereby obtained a scaffold material. With respect to the scaffold material obtained by treating in the metallic Schale, the surface layer positioned at an upper opening side of the metallic Schale is referred to as the "upper surface layer" and the surface layer positioned at the bottom surface side of the metallic Schale is referred to as the "lower surface layer".

### (Evaluation of scaffold material)

### (1) Appearance

Each of the obtained scaffold materials was cut in the thickness direction using a microtome blade, and the cross section was photographed with a digital microscope (VHX-900, produced by KEYENCE CORPORATION.).

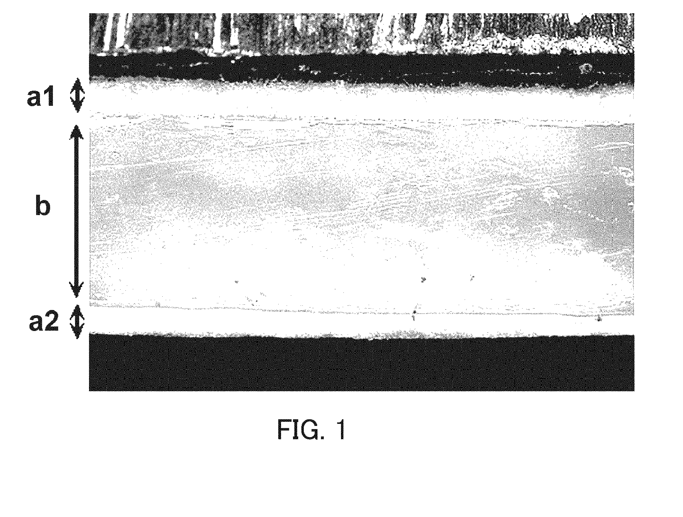

In FIG. 1, shown is a cross sectional photograph of the scaffold material produced under the following conditions: the immersion time was 10 seconds and the freezing treatment temperature was -50°C. In FIG. 1, a1 is an upper surface layer, a2 is a lower surface layer, and b is a core layer. As shown in FIG. 1, in the scaffold material of Example 1, the thickness of the surface layer a1 and the thickness of the surface layer a2 were uniform, and the thickness of the core layer b was uniform. Also with respect to the scaffold material obtained under the immersion time and the freezing treatment temperature different from those described above, as in the case of above, it was confirmed that the thicknesses of the surface layers and the thickness of the core layer were uniform.

### (2) Thicknesses of surface layers and core layer

From each of the cross sectional photographs of the scaffold materials, the thickness of the core layer and the thicknesses of the surface layers were measured. The thickness of the core layer b was measured at 10 points per piece (n=2). With respect to the surface layers, the thickness of the upper surface layer a1 and the thickness of the lower surface layer a2 were each measured at 5 points per piece (n=4). Then, the average thickness of each was calculated.

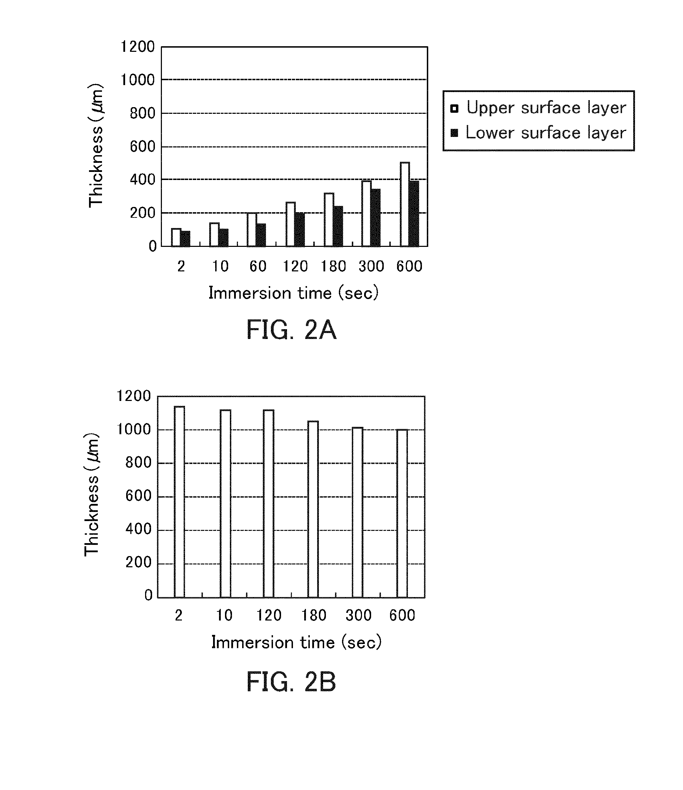

FIG. 2(A) shows a graph showing the relationship between the immersion time of the polymer substrate and the thicknesses of the upper surface layer and the lower surface layer of the scaffold material. FIG. 2(B) shows a graph showing the relationship between the immersion time and the thickness of the core layer of the scaffold material. In each graph of FIGs. 2(A) and 2(B), the horizontal axis indicates the immersion time (sec) and the vertical axis indicates the thickness (µm). As shown in FIGs. 2(A) and 2(B), as the immersion time was increased, the thickness of the core layer was decreased, the thicknesses of the both upper and lower surface layers were increased (about 170 to 500 µm), and the entire thickness of the scaffold material was increased. This result showed that the thicknesses of the surface layers, the core layer, and the scaffold material can be adjusted according to the immersion time to the solvent.

### (3) Pore size of surface layer

Each of the obtained scaffold materials was cut in the thickness direction in the same manner as described above. Then, platinum was vapor-deposited on each of the pieces using an ion sputter (E-1010, produced by Hitachi, Ltd.). With respect to this vapor-deposited piece, the surface of the upper surface layer (a1) was photographed using a SEM (Type-N, produced by Hitachi, Ltd.).

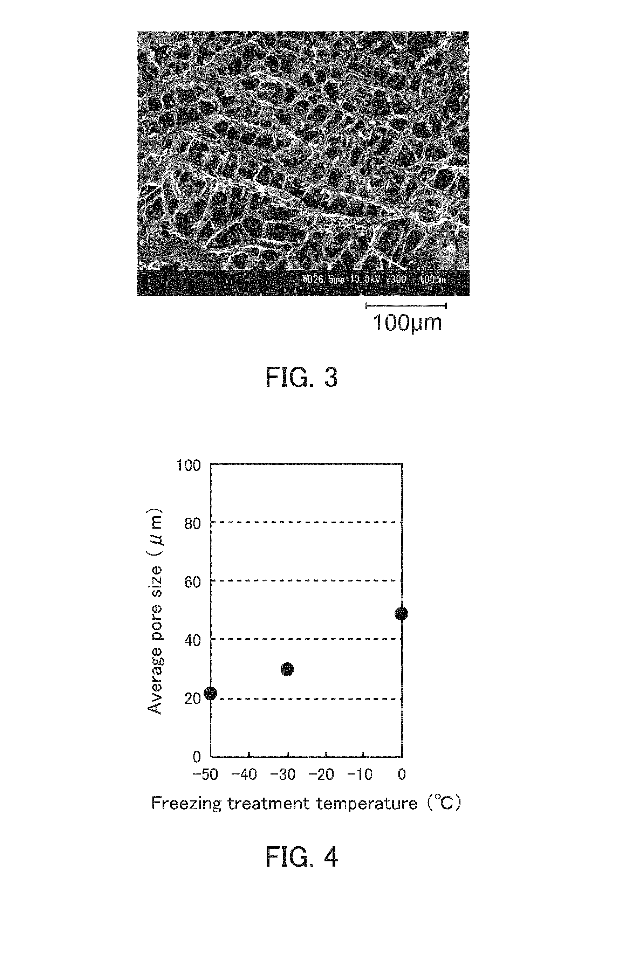

FIG. 3 shows a scanning electron micrograph of the scaffold material produced under the following conditions: the immersion time was 10 seconds and the freezing treatment temperature was -50°C. FIG. 3 is a photograph of the upper surface layer of the scaffold material. In FIG. 3, the length of the bar indicated at the bottom right of the photograph represents 100 µm. As shown in FIG. 3, it was confirmed that the upper surface layer of the scaffold material had pores of uniform pore sizes. Further, it was confirmed that the lower surface layer of the scaffold material had pores of uniform pore sizes. Also with respect to the scaffold material obtained under the immersion time and the freezing treatment temperature different from those described above, as in the case of above, it was confirmed that each of the layers has pores of uniform pore sizes.

### (4) Relationship between pore size and freezing treatment temperature

The image obtained in (3) was analyzed using image analysis software (Image J), and the average pore size was calculated.

FIG. 4 shows a graph showing the relationship between the freezing treatment temperature of the polymer substrate and the average pore size (n=3). In FIG. 4, the horizontal axis indicates the freezing treatment temperature (°C) and the vertical axis indicates the average pore size (µm) of the upper surface layer. As shown in FIG. 4, as the freezing treatment temperature was increased, the average pore size was increased. This result showed that the pore size can be adjusted according to the freezing treatment temperature.

### (Example 2)

In Example 2, cells were disseminated in a scaffold material and cultured for 18 days, and the proliferation was ascertained.

### (Production of scaffold material)

The scaffold material was produced in the same manner as in Example 1 except that the immersion time of the polymer substrate was 10 seconds and the freezing treatment temperature was ―50°C. The scaffold material obtained in this manner was cut into 5 mm × 5 mm pieces, immersed in 99.5 v/v% ethanol overnight, and then dried.

### (Dissemination of cell)

First, Chinese hamster lung-derived fibroblast (V79) was added to a MEM culture medium containing 10% fetal bovine serum (FBS) such that the resultant mixture has a concentration of 7.7 × 10⁵ cell/mL, and thereby prepared a cell suspension. The dried scaffold material was introduced into a 30mL syringe, and the syringe was filled with 10 mL of the cell suspension. A unidirectional valve was attached to the tip of the syringe, the pressure in the syringe was reduced by pulling a plunger, and the air in the scaffold material was removed. By tapping the syringe lightly, the air in the syringe was removed. This air removal operation was repeated for 3 times, 10 mL of the cell suspension was replaced, and the air removal operation was repeated for 3 times in the same manner as described above. The scaffold material impregnated with the cell suspension and a MEM culture medium containing 10v/v% FBS were introduced in a culture flask and cultured for 18 days. During the culture, static culture was performed in the first day and shake culture was performed on the rest of the days. During the culture, the culture medium was changed on a cell proliferation evaluation day that will be described below.

### (Evaluation of cell proliferation)

With respect to the scaffold material (n=10), the cell proliferation was evaluated as described below 1, 4, 8, 11, 15, and 18 days after the start of the culture. That is, first, the scaffold material was washed lightly with a phosphate buffer solution (PBS). Subsequently, the scaffold material was immersed in 1 mL of 0.05 w/v% trypsin and treated at 37°C for 30 minutes to detach cells from the scaffold material. The obtained solution containing detached cells was added to 9 mL of an electrolytic solution (trade name: ISOTON, produced by Beckman Coulter, Inc.), and the number of cells was counted using a Coulter counter. The counting was performed with respect to suspended solids of 10 µm or more.

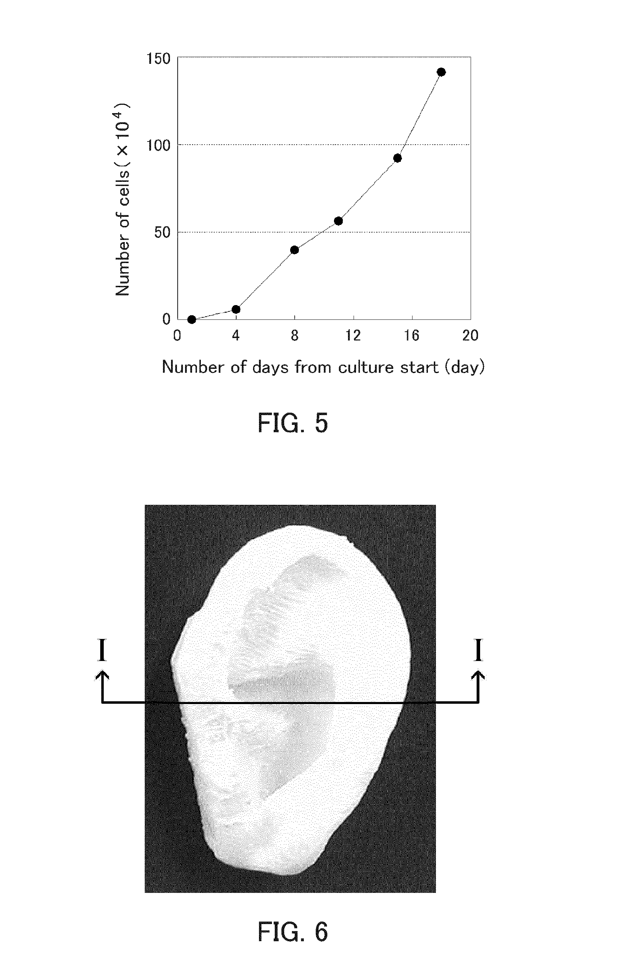

FIG. 5 shows a graph showing the result of measurement of the number of cells. In FIG. 5, the horizontal axis indicates days from the start of the culture and the vertical axis indicated the number of cells (×10⁴). As shown in FIG. 5, the number of cells increased as the number of days of culture increased. From this result, the proliferation of cells disseminated in a scaffold material was confirmed.

After culturing in the same manner as described above, the scaffold material was subjected to Giemsa staining. As a result, staining was confirmed on the surface layer of the scaffold material. Further, since the density of the stain was increased over time according to the number of days of culture, it was confirmed that the cell was proliferated in the surface layer.

### (Example 3)

In Example 3, an auricle shaped scaffold material was produced.

### (Production of polymer substrate)

The P (LA/CL) used in Example 1 was heated to 200°C and poured into a silicon mold having a cavity in a shape of an auricle. The silicon mold was immersed in ice water and the P (LA/CL) was cured by cooling. The cured P (LA/CL) was taken out from the silicon mold and was used as an auricle shaped polymer substrate.

### (Production of scaffold material)

The scaffold material was produced in the same manner as in Example 1 except that the immersion time of the polymer substrate was 1 to 2 seconds and the temperature of the cooling rack (freezing treatment temperature) was -80°C.

### (Evaluation of shape of scaffold material)

The shape of the obtained scaffold material was observed. Further, the scaffold material was cut using a microtome blade, and the cross section was photographed with a digital microscope (VHX-900, produced by KEYENCE CORPORATION.).

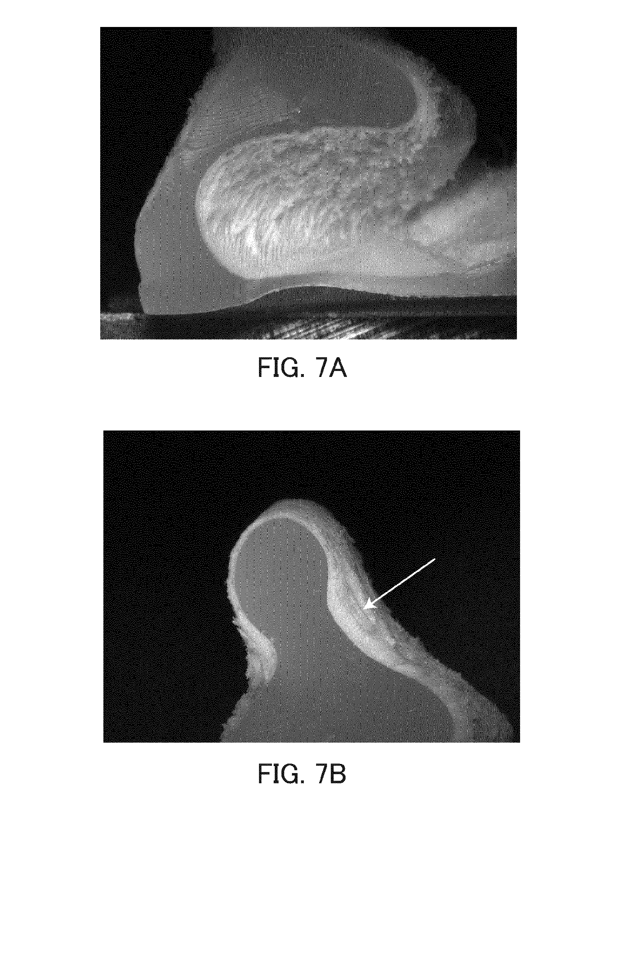

FIG. 6 shows a photograph of the appearance of the scaffold material. FIGs. 7(A) and (B) show cross sectional photographs of the scaffold material. FIG. 7(A) is a partial photograph of the cross section taken along the line I-I of the scaffold material in FIG. 6; and FIG. 7(B) is a partial photograph of the cross section of another site. As shown in FIG. 6, the obtained scaffold material has a complicated auricle structure including the helix, scapha, concha, ear lobe, and the like; and the scaffold material was formed in substantially the same shape as the polymer substrate. Further, as shown in FIG. 7, even when the porous surface layer (for example, the layer indicated by the arrow in FIG. 7(B)) was formed on the entire surface of the scaffold material and the scaffold material was in a complicated shape having various curved surfaces, the thickness of the surface layer was substantially uniform. In this manner, according to the production method of the present invention, a scaffold material in a complicated shape can be formed easily without forming an adhesive layer.

### Industrial Applicability

The porous member of the present invention is a porous member formed not by the paste method. Therefore, unlike the porous member obtained by the paste method, for example, the porous member of the present invention does not include an adhesive layer formed by bonding between the core layer and the porous surface layer. Such a porous member can be produced, for example, by the method of producing a porous member of the present invention. In other words, according to the production method of the present invention, the surface of the polymer substrate can be made porous easily by simply immersing the polymer substrate in the solvent and then freeze-drying the polymer substrate. In this manner, the porous member of the present invention in which the porous surface layer is formed integrally on the surface of the core layer can be produced. Further, according to the production method of the present invention, the scaffold material including the porous surface layer and the core layer as a single member can be formed easily regardless of the shape of the polymer substrate. In this manner, since a desired shaped porous member that does not include an adhesive layer can be provided easily according to the present invention, the present invention is very effective for provision of a scaffold material or the like in the field of regenerative medicine, for example.

## Claims

1. A porous member having a porous surface, comprising:
a core layer; and
a porous surface layer, wherein
the core layer and the surface layer are composed of the same polymer raw material,
the surface layer is integrally formed on a surface of the core layer, and the porous member does not comprise an adhesive layer between the core layer and the surface layer.

2. The porous member according to claim 1, wherein the surface layer has a thickness from 10 to 1000 µm.

3. The porous member according to claim 1, wherein the core layer is non-porous.

4. The porous member according to claim 1, wherein the core layer is porous and the core layer has a porosity that is relatively lower than a porosity of the surface layer.

5. The porous member according to claim 1, wherein the polymer raw material includes a biodegradable polymer.

6. The porous member according to claim 5, wherein the biodegradable polymer is a copolymer of lactide and caprolactone.

7. The porous member according to claim 1, wherein the porous member is in a shape of a plate, a column, or a tube.

8. The porous member according to claim 1, wherein the porous member takes an entire or a partial shape of a biological tissue.

9. The porous member according to claim 1, wherein the porous member is intended for use in a biomaterial.

10. The porous member according to claim 1, wherein the porous member is configured for use in a scaffold material for a cell, a stent, or an adhesion-preventing material.

11. The porous member according to claim 1, wherein the porous member is configured for use in a bioprosthesis.

12. A method for making a surface of a polymer substrate porous, comprising the following processes (A) and (B):
(A) immersing the polymer substrate in a solvent capable of dissolving the polymer substrate; and
(B) freeze-drying the immersed polymer substrate.

13. The method according to claim 12, wherein, in the process (B), the polymer substrate is subjected to freezing treatment in a state where it is immersed in the solvent.

14. The method according to claim 12, wherein a polymer raw material that constitutes the polymer substrate includes a biodegradable polymer.

15. The method according to claim 14, wherein the biodegradable polymer is a copolymer of lactide and caprolactone.

16. The method according to claim 12, wherein the solvent contains 1,4-dioxane.

17. The method according to claim 12, wherein, in the process (A), a pore size of the surface layer to be formed is controlled by adjusting an immersion time of the polymer substrate in the solvent.

18. The method according to claim 12, wherein, in the process (B), a pore size of the surface layer to be formed is controlled by adjusting a temperature of freezing treatment of the polymer substrate.

19. The method according to claim 12, wherein, in the process (A), a thickness of the surface layer to be formed is controlled by adjusting an immersion time of the polymer substrate in the solvent.

20. The method according to claim 12, wherein, in the process (B), the polymer substrate is cooled at a constant speed.

21. The method according to claim 12, wherein the polymer substrate is a non-porous substrate.

22. The method according to claim 12, wherein the polymer substrate is in a shape of a plate, a column, or a tube.

23. The method according to claim 12, wherein the polymer substrate takes an entire or a partial shape of a biological tissue.

24. A method of producing a porous member, wherein a surface of a polymer substrate is made porous by the method according to claim 12.

25. A porous member produced by the method according to claim 24.
